# EUROPEAN PATENT SPECIFICATION

(11) **EP 1 263 464 B1**
(45) Date of publication and mention of the grant of the patent: **31.10.2012**
(21) Application number: 01956188.5
(22) Date of filing: 08.03.2001
(51) Int. Cl.: C07K 16/22, A61K 39/395, A61P 13/12

(54) **USE OF ANTAGONIST ANTI-TGF-BETA ANTIBODIES TO TREAT OR TO PREVENT LOSS OF RENAL FUNCTION**
VERWENDUNG VON ANTAGONISTISCHEN ANTI-TGF-BETA ANTIKÖRPERN ZUR BEHANDLUNG ODER ZUR VORBEUGUNG VON VERLUST AN NIERENFUNKTION
EMPLOI D'ANTICORPS ANTAGONISTES CONTRE LE TGF-BETA DANS LE TRAITEMENT OU LA PRÉVENTION DE LA PERTE DE LA FONCTION RENALE

(30) Priority: 09.03.2000 US 188060 P
(43) Date of publication of application: 11.12.2002
(73) Proprietor: GENZYME CORPORATION, Cambridge, Massachusetts 02139-1562 (US); MCW Research Foundation, Milwaukee, Wisconsin 53226 (US)
(72) Inventor: LEDBETTER, Steven, R., Westborough, MA 01581 (US); ROMAN, Richard, J., Brookfield, WI 53005 (US)
(74) Representative: Adams, Harvey Vaughan John
(86) International application number: PCT/US2001/007473
(87) International publication number: WO 2001/066140

(56) References cited:
- WO-A-91/19513
- WO-A-99/61040
- DAHLY A J ET AL: "Chronic anti-TGF-beta therapy improves blood pressure and renal function in Dahl S rats." FASEB JOURNAL, vol. 14, no. 4, 15 March 2000 (2000-03-15), page A133 XP002173612 Annual Meeting of Professional Research Scientists: Experimental Biology 2000;San Diego, California, USA; April 15-18, 2000 ISSN: 0892-6638

## Description

### Field of the Invention

The present invention is in the fields of molecular biology and renal (patho)biology The present invention relates to the preparation of pharmaceutical compositions for use in novel methods for treating or preventing loss of renal function by administering to an individual an effective amount of a TGF-β antagonist, wherein said TGF-β antagonist is an antibody or antibody fragment that is directed against one or more isoforms of TGF-β.

### Background of the Invention

In vertebrate animals, the kidney functions to reabsorb water and to concentrate and to remove waste metabolites from the circulatory system, as well as regulate the pH, salt balance, and volume of the blood. Acute or chronic loss of kidney function, due to injury, disease, or some intrinsic disorder, can cause a variety of systemic complications. End stage renal failure, treatable only by dialysis or organ transplant, can lead to death.

The kidney is macroscopically divided into three major regions, the (outer) renal cortex, the (inner) renal medulla, and the renal pelvis. Microscopically, the kidney is composed of millions of unitary functional nephrons embedded within vast arrays of capillary beds. Each nephron has its own blood supply. The structural nephron is a "U" shaped renal tubule, which spans the cortex and medulla of the kidney. The nephron is composed of the Bowman's capsule, which encompasses a glomerular tuft of capillaries, or glomerulus, and is located in the renal cortex. The proximal convoluted tubule leads from the Bowman's capsule to the descending limb, to the loop of Henle, and then to the ascending limb, all of which are located in the renal medulla and intimately associated with the vasa recta, the region of the peritubular capillaries that resides within the renal medulla. The ascending limb leads to the distal convoluted tubule, located in the cortex, which empties into collecting ducts that terminate at the renal pelvis.

Three physiological processes are involved in proper kidney function; pressure filtration, selective reabsorption, and tubular secretion, all of which serve to conserve water and rid the body of nitrogenous waste products and salts before they reach toxic levels.

Pressure filtration occurs in the cortex, across the thin walls of capillaries in the glomerulus. Pressure filtration refers to the process wherein large molecules and formed elements of the blood are retained within the circulatory system but normal glomerular blood pressure (typically about 60 mm Hg) force water and small molecules (salts and various nutrient and waste compounds) into the Bowman's capsular space, which is continuous with the lumen of the proximal tubule.

Selective reabsorption occurs within the renal medulla as various components (e.g., water, glucose, sodium, and chloride) are actively and passively reabsorbed from the thin ascending and descending limb of the loop of Henle, and the thick ascending limb of the loop of Henle, into the vasa recta. Selective reabsorption occurs partially as a result of a complex countercurrent system involving vasa recta capillaries and renal tubules in the medulla, whereby active reabsorption of sodium ions causes a concomitant passive reabsorption of chloride ions and water, creating a gradient of osmolality.

Tubular secretion is a process whereby various blood components (e.g., hydrogen and ammonium ions, creatine, and various drugs such as penicillin) are actively secreted from the circulatory system into the proximal and distal convoluted tubules. Because of the active transport of hydrogen ions, tubular secretion also regulates blood pH.

Proper kidney function thus produces a final waste product that contains substances removed from the circulatory system due to pressure filtration through the glomerulus and tubular secretion into the proximal and distal convoluted tubules of the renal cortex, and the reabsorption of (primarily) water occurring in the descending limb of the loop of Henle, and the medullary collecting duct due to the concentrating mechanism of the renal medulla.

Because of the vital function the kidney performs in maintaining proper body fluid homeostasis, loss of renal function represents a life-threatening event. Typically, insults to the kidney initiate a wound repair response. Part of this protective mechanism involves tissue repair and remodeling. If tissue repair is not properly regulated, however, fibrosis occurs. Many renal diseases and disorders, therefore, exhibit a concomitant fibrosis of the kidney.

Current research concerning progression and treatment of kidney diseases and disorders has focussed on mechanisms to arrest fibrosis within the renal cortex. Renal blood flow in humans is the highest in the body, relative to organ weight, and represents one fourth of total cardiac output. Most renal blood flow is directed to the cortex to optimize glomerular filtration. Because of the role the glomerulus plays as the site of pressure filtration, conventional wisdom has directed investigators to research and to develop therapeutics that would halt, or at least slow fibrosis of the glomerulus. TGF-β has been a target in the treatment of glomerular fibrosis, and the use of TGF-β antagonists as potential therapeutics to slow the progression of cortical fibrosis has been investigated extensively. The association between cortical fibrosis and kidney dysfunction has not been demonstrated, however.

TGF-β is a member of a superfamily of polypeptides that control development and tissue homeostasis in organisms as diverse as drosophila and humans (Grande, 1997). TGF-β displays ubiquitous and diverse biologic functions ranging from energy production in mitochondria; to regulation of vascular tone; to cellular differentiation, proliferation and apoptosis. Nevertheless, TGF-β is best known as a cytokine responsible for activating extracellular matrix production associated with wound repair.

Extensive research has shown that TGF-β plays an important role in inducing increased synthesis and decreased metabolism of extracellular matrix proteins (e.g., fibronectin, collagens and proteoglycans) in glomeruli leading to glomerular diseases such as glomerular sclerosis. A large latent TGF-β complex, composed of latency associated peptides (LAP, which are in fact C-terminal domains of precursor TGF-β's), TGF-β, and a latent TGF-β binding protein (LTBP), is found in glomeruli (Mackay et al., 1992).

Elevated TGF-β serum levels have been associated with glomerular sclerosis and fibrosis (Bottinger and Kopp, 1998). Ruiz-Torres et al. (1998) have suggested that TGF-β acts as a fibrogenic growth factor, which is, at least partially, responsible for interstitial fibrosis of the renal cortex associated with aging, and that treatment with captopril, an angiotensin-converting enzyme inhibitor, may slow the progression of cortical interstitial lesions (Also see Wolf, 1998).

Border et al. (1990, 1992a, 1992b, 1994; See also Border and Ruoslahti, 1991; Ruoslahti et al., 1993; Ruoslahti & Border, 1993; Ruoslahti et al., 1998) has reported that glomerulonephritis, an inflammation of the kidney characterized by the accumulation of extracellular matrix within damaged glomeruli, is associated with increased production and activity of TGF-β1, and that administration of a TGF-β1 antagonist can suppress increased production of extracellular matrix in glomeruli. Others have reported that TGF-β antagonists may be used to suppress TGF-β induced extracellular matrix deposition and fibrosis (Dasch et al., 1996; Ferguson et al., 1997; Gotwals et al., 1998; Logan and Baird, 1999; Ruoslahti et al., 1995, 1996, 1997, 1998; Segarini et al., 1997). WO 91/19513 discloses the potential use of TGF-β antagonists for the treatment of hypotension in acute renal failure.

Fibrosis, *per se,* is not the ultimate cause of renal dysfunction, however. Indeed, although the fibrogenic process is believed to be a factor in the obliteration of renal tubules, the precise connection between renal fibrosis and the decline in renal function is unknown (Eddy, 1996). Renal tubular damage, in fact, occurs early in the course of progressive renal disease. Suspected causative agents of tubular damage include tubular obstruction, lysosomal enzymes, reactive oxygen metabolites, complement proteins, and ischemia (Eddy, 1994).

Despite the extensive research efforts detailed above, effective treatment and prophylaxis of renal dysfunction does not exist. Anti-TGF-β agents have been suggested as potential therapeutics in the treatment of fibrosis of the renal cortex, which may exacerbate loss of kidney function by obliteration of glomerular capillaries. These agents have not heretofore been reported to have any impact on renal function, however. Moreover, the precise mechanisms of initial and progressive loss in renal function are unclear. There is a need in the art, therefore, to understand the ultimate causes of renal dysfunction and to discover and to develop effective therapeutics useful for directly treating and preventing loss of renal function.

### Summary of the Invention

The present invention is based upon the discovery that TGF-β antagonists are useful for treating and preventing loss of kidney function occurring in the context of acute and chronic kidney disease. Although other studies have suggested the use of anti-TGF-β agents to treat a variety of fibroproliferative disorders (including those of the renal cortex), none has provided any demonstration that reduction in fibrosis would have any impact on the associated disease, *per se.* Disclosed herein is the first demonstration that antagonism of TGF-β effectively slows the progression of kidney damage, e.g., by preventing loss of renal vascular circulation, reducing tubular injury of the renal medulla, and preventing systemic hypertension.

This discovery marks a significant departure from the current state of the art concerning therapeutic renal research, which has focused on the treatment of (secondary and symptomatic) cortical fibrosis. Cortical fibrosis (e.g., glomerular sclerosis) serves to exacerbate loss of kidney function by physically obstructing the vasculature and tubules of the renal cortex. No evidence exists however, that renal dysfunction can be prevented by treatment for cortical fibrosis.

In contrast, the present invention demonstrates the importance of proper medullary function, and specifically the role of hypoxic injury of medullary tubules in the onset and progression of diseases and disorders of the kidney. This discovery supports a view that compromised renal circulation is fundamental to the onset of acute and chronic kidney disorders. Renal health is ultimatefy a function of renal circulation and tubular integrity. Renal fibrosis is viewed as a separate (and perhaps secondary) consequence of (and with subsequent aggravating effects upon) reduced capillary and tubular function. As a result, effective treatments for renal disease need to focus on maintaining the circulatory and tubular integrity of the kidney.

Although TGF-β is best known as a cytokine responsible for activating extracellular matrix production associated with wound repair, and although it remains the premier fibrogenic cytokine of study concerning glomerular fibrosis in particular, TGF-β displays ubiquitous and diverse biologic functions. The present invention teaches that TGF-β plays a significant role in renal function *per se,* separate from its role in cortical fibroproliferative conditions, and demonstrates that TGF-β antagonists act as effective therapeutics, preventing loss of renal function by maintaining an adequate circulation, especially in the renal medulla.

It is therefore an object of the present disclosure to provide a method for treating a disease or disorder of the kidney comprising administering to an individual afflicted with such disease a pharmaceutically effective amount of a TGF-β antagonist.

In a related aspect of the present disclosure, it is an object to provide the use of a TGF-β antagonist for the preparation of a pharmaceutical composition useful for treating a disease or disorder of the kidney as described herein.

It is a further object of the disclosure that a TGF-β antagonist is used to maintain renal function, or to slow, to halt, to prevent, or to reverse loss of renal function, specifically of the renal medulla, and more specifically to preserve the vascular and tubular integrity of the renal medulla. Preferred instances of the present disclosure include administering a pharmaceutically effective amount of a TGF-β antagonist to maintain and to regulate desirable levels for systemic blood pressure, medullary blood flow, or to reduce or to inhibit proteinuria, medullary fibrosis, medullary ischemia, systemic hypertension, and/or hypoxic tubular injury and necrosis of the medulla sufficient to maintain renal function.

TGF-β antagonists of the present disclosure include any molecule that is able to decrease the amount or activity of TGF-β, either within a cell or within a physiological system. TGF-β antagonists of the present disclosure also include any nucleic acid sequence that encodes a molecule capable of decreasing the amount or activity of TGF-β. Preferably, TGF-β antagonists include: antibodies directed against one or more isoforms of TGF-β; TGF-β receptors and soluble fragments thereof; antibodies directed against TGF-β receptors; latency associated peptide; large latent TGF-β; TGF-β inhibiting proteoglycans such as fetuin, decorin, biglycan, fibromodulin, lumican and endoglin; somatostatin; mannose-6-phosphate; mannose-1-phosphate; prolactin; insulin-like growth factor II; IP-10; arg-gly-asp containing peptides; extracts from plants, fungi, or bacteria; antisense oligonucleotides; proteins involved in TGF-β signating, including SMADs, MADs, Ski, Sno; and any mutants, fragments or derivatives of the above-identified molecules that retain the ability to inhibit the activity of TGF-β. More preferably the TGF-β antagonist is a human or humanized monoclonal antibody that blocks TGF-β binding to its receptor (or fragments thereof such as F(ab)₂ fragments, Fv fragments, single chain antibodies and other forms of "antibodies" that retain the ability to bind to TGF-β. Most preferred, the monoclonal antibody is humanized form of the murine monoclonal antibody obtained from hybridoma 1D11.16 (ATCC Accession No. HB 9849).

Based on the disclosure contained herein, the present invention provides for the use of a TGF-β antagonist for the preparation of a pharmaceutical composition for reducing systemic hypertension, maintaining medullary blood flow, reducing the loss of renal medullary function, or reducing hypoxic tubular injury, in an individual suffering from or potentially susceptible to loss of kidney function, wherein said TGF-β antagonist is an antibody or antibody fragment that is directed against one or more isoforms of TGF-β.

In a related aspect, the present invention provides for a TGF-β antagonist, for use in reducing systemic hypertension, maintaining medullary blood flow, reducing the loss of renal medullary function, or reducing hypoxic tubular injury, in an individual suffering from or potentially susceptible to loss of kidney function, wherein said TGF-β antagonist is an antibody or antibody fragment that is directed against one or more isoforms of TGF-β.

In a further aspect, the present invention provides for the use of a TGF-β antagonist for the preparation of a pharmaceutical composition for treating or slowing the progression of kidney damage associated with a disease or disorder of the kidney selected from the group consisting of obstructive nephropathy, polycystic kidney disease, nephrosclerosis, and nephrocalcinosis, wherein said TGF-β antagonist is an antibody or antibody fragment that is directed against one or more isoforms of TGF-β.

In a related aspect, the present invention provides for a TGF-β antagonist, for use in treating or slowing the progression of kidney damage associated with a disease or disorder of the kidney selected from the group consisting of obstructive nephropathy, polycystic kidney disease, nephrosclerosis, and nephrocalcinosis, wherein said TGF-β antagonist is an antibody or antibody fragment that is directed against one or more isoforms of TGF-β.

Further aspects and embodiments of the present invention are set out in the appended claims.

### Brief Description of the Drawings

**FIG. 1** provides comparative histological light micrographs of PAS-stained glomeruli (panels A & B) and renal medulla (panels C & D) sections prepared from untreated (panels A & C) and anti-TGF-β treated (panels B & D) Dahl S rats fed a high salt diet.
**FIG. 2** depicts early intervention effects of anti-TGF-β Ab therapy on protein (a) and albumin excretion (b) in Dahl S rats fed a high salt diet.

### Detailed Description of the Invention

Disclosed herein is the first report describing the effective use of TGF-β antagonists to slow the progression of kidney damage that would otherwise occur during chronic kidney disease. As demonstrated herein, TGF-β antagonists are useful to prevent and to reduce loss of renal vascular circulation, tubular injury of the renal medulla, and systemic hypertension. Based upon previous studies of the development of glomerulosclerosis in a hypertensive animal model, conventional thought in the art is that cortical damage is the proximate cause of loss of renal function. Presented herein for the first time is the unexpected discovery that indices of cortical hypertrophy and glomerular injury between a hypertensive animal group treated with a TGF-β antagonist and an untreated control group were not significantly different (despite a significant reduction of proteinuria exhibited in test group). Anti-TGF-β treated animals unexpectedly exhibited, however, significantly lower mean arterial pressure, significantly lower fibrosis of the vasa recta, significantly lower medullary tubular injury, significantly lower medullary tubular necrosis, and significantly higher medullary blood flow compared to control animals.

As described earlier, renal function is achieved through the processes of pressure filtration, selective reabsorption, and tubular secretion, all of which are highly dependent upon the integrity of each nephron in close association with the surrounding vasculature. The countercurrent system of the renal medulla, which is responsible for the process of selective reabsorption, occurs within a localized low oxygen (hypoxic) medullary environment. An exacting match of oxygen supply and demand by precise regulation of the medullary blood flow and tubular system is critical to proper renal function (Brezis and Rosen, 1995). In humans, medullary partial pressure of oxygen ranges from about 10 to 20 mm Hg, while cortical O₂ partial pressure is about 50 mm Hg (Brezis et al., 1991, 1994a, and 1994b). Medullary hypoxia poses a constant threat to cellular integrity, however, and renders the medulla highly susceptible to injury, especially if the delicate oxygen balance within the medulla is impaired.

Medullary hypoxic injury is typically characterized by medullary ischemia and tubular necrosis, especially in the high metabolically active thick ascending loop of Henle. In addition, anaerobic conditions and anoxia in the medulla facilitate fibrosis of the vasa recta. The present discovery highlights the importance of proper medullary function, and specifically the role medullary hypoxic injury plays in the onset and progression of diseases and disorders of the kidney. This discovery supports a view that compromised renal circulation and tubular injury is fundamental to the onset of acute and chronic kidney disorders.

The present disclosure is directed to a method for treating or slowing the progression of kidney damage associated with a disease or disorder of the kidney comprising administering to an individual suffering from said disease or disorder a pharmaceutically effective amount of a TGF-β antagonist The present disclosure is also directed to use of a TGF-β antagonist for preparation of a pharmaceutical composition useful for treating or slowing the progression of kidney damage associated with a disease or disorder of the kidney as described herein. In one instance, the disease or disorder of the kidney is chronic. In another instance, the disease or disorder of the kidney is acute.

Diseases or disorders of the kidney include any acute or chronic disease or disorder that compromises renal circulation, causes tubular injury, or otherwise causes a diminution in renal function. A wide variety of diseases or disorders can induce renal pathologies, including rheumatic/immunologic disorders, genetic/metabolic disorders, hematologic/oncologic disorders, infectious disorders, radiation injury, renal surgery, lithotripsy, or drug- or toxin-inducedlnephrotoxic disorders. Such diseases or disorders include, but are not limited to, diabetic (type I and type II) nephropathy, obstructive nephropathy, hereditary renal disease (e.g., polycystic kidney disease, medullary sponge kidney, horseshoe kidney), glomerulonephritis, nephrosclerosis, nephrocalcinosis, systemic lupus, Sjogren's syndrome, hypertension, tubulointerstitial nephropathy, renal tubular acidosis, renal tuberculosis, or renal infarction.

As used herein, "treating or slowing the progression of kidney damage" generally refers to any process that functions to slow, to halt (including stopping initial onset), or to reverse loss of renal function.

Loss of renal (or kidney) function, as used herein, refers to any physiological disruption or dysfunction of normal renal function of an animal. For the purposes of this disclosure, including the invention, mere structural abnormalities (e.g., fibrosis) of the kidney are not considered, *per se,* kidney dysfunctions, or a disease or disorder of the kidney. Loss of kidney function specifically refers to the diminution of pressure filtration, selective reabsorption, or tubular secretion of the kidney. Loss of renal function includes, but is not limited to, medullary hypoperfusion; medullary hypoxia; including hypoxic tubular injury, tubular necrosis, formation of protein casts and tubular obstruction, or other manifestations that reduce tubular flow; as well as manifestations that reduce medullary blood flow such as ischemia and other vasa recta injury.

As used herein, a "pharmaceutical composition" refers to any composition that contains a pharmaceutically effective amount of one or more active ingredients (e.g., a TGF-β antagonist) in combination with one or more pharmaceutical carriers and/or additives. Determination of suitable pharmaceutical carriers and/or additives useful for a pharmaceutical composition, as well as the form, formulation, and dosage of such composition, is well within the ability of those skilled in the art (see, for example, Remington's Pharmaceutical Sciences, Mack Publishing Co.). Carriers and/or additives may include but are not limited to: excipients; disintegrators; binders; thickeners, lubricants; aqueous vehicles; oily vehicles; dispersants; preservatives; and isotonizing, buffering, solubilizing, soothing and/or stabilizing agents. The proportion of active ingredient(s) in a pharmaceutical composition of the present disclosure can be appropriately determined by a person of skill in the art based upon, e.g., the individual, the individual's age and body weight, the individual's clinical status, administration time, dosage form, method of administration, and combination of active components, among other factors. Preferably, the pharmaceutical composition of the present disclosure is low in toxicity and can safely be used in vertebrates, more preferably mammals, and most preferably humans.

As used herein, a "pharmaceutically effective amount" is an amount effective to achieve the desired physiological result in a subject. Specifically, a pharmaceutically effective amount of a TGF-β antagonist is an amount sufficient to decrease the quantity or activity of TGF-β for a period of time sufficient to ameliorate one or more of the pathological processes associated with loss of renal function. The effective amount may vary depending on the specific TGF-β antagonist selected, and is also dependent on a variety of factors and conditions related to the subject to be treated and the severity of the disorder (for example, the age, weight and health of the patient as well as dose response curves and toxicity data). The determination of a pharmaceutically effective amount for a given agent is well within the ability of those skilled in the art.

"Administration" to an individual is not limited to any particular delivery system and may include, without limitation, parenteral (including subcutaneous, intravenous, intramedullary, intraarticutar, intramuscular, or intraperitoneal injection) rectal, topical, transdermal or oral (for example, in capsules, suspensions or tablets). Administration to an individual may occur in a single dose or in repeat administrations, and in any of a variety of physiologically acceptable salt forms, and/or with an acceptable pharmaceutical carrier and/or additive as part of a pharmaceutical composition (described earlier). Once again, physiologically acceptable salt forms and standard pharmaceutical formulation techniques are well known to persons skilled in the art (see, for example, Remington's Pharmaceutical Sciences, Mack Publishing Co.). Administration of a TGF-β antagonist to an individual may also be by means of gene therapy, wherein a nucleic acid sequence encoding the antagonist is administered to the patient *in vivo* or to cells *in vitro,* which are then introduced into a patient, and the antagonist is produced by expression of the product encoded by the nucleic acid sequence. Methods for gene therapy to deliver TGF-β antagonists are also well known to those of skill in the art (See, for example, Border, 1996).

As used herein, "individual" refers to any vertebrate suffering from or potentially susceptible to loss of kidney function, including any disease or disorder of the kidney as defined herein. Dahl S rats, well known and used in the art for the study of salt-sensitive hypertension and renal dysfunction, are specifically described herein as a representative animal model only, and should not be construed as limiting to the scope of this disclosure, including the invention. Previous studies indicate that Dahl S rats exhibit many traits associated with salt-sensitive hypertension in humans. Dahl S rats are salt-sensitive, insulin-resistant and hyperlipidemic and have a low renin form of hypertension that is refractory to converting enzyme inhibitors but responds well to treatment with Ca⁺⁺ channel blockers and diuretics. Dahl S rats also rapidly develop severe proteinuria, glomerulosclerosis and tubulointerstitial renal disease during the development of hypertension that progresses to endstage renal disease. The glomerular lesions that develop resemble those seen in human patients with hypertension- and diabetes-induced glomerulosclerosis.

As used herein, "TGF-β" refers to all isoforms of TGF-β. There are currently 5 known isoforms of TGF-β (1-5), all of which are homologous (60-80% identity) and all of which form homodimers of about 25 kD, and act upon common TGF-β cellular receptors (Types I, II, and III). The genetic and molecular biology of TGF-β is well known in the art (see, for example, Roberts, 1998; Wrana, 1998).

As used herein, a "TGF-β antagonist" is any molecule that is able to decrease the amount or activity of TGF-β either within a cell or within a physiological system. Preferably, the TGF-β antagonist acts to decrease the amount or activity of a mammalian TGF-β1, 2, or 3. For example, a TGF-β antagonist may be a molecule which inhibits expression of TGF-β at the level of transcription, translation, processing, or transport; it may affect the stability of TGF-β or conversion of the precursor molecule to the active, mature form; it may affect the ability of TGF-β to bind to one or more cellular receptors (e.g., Type I, II or III); or it may interfere with TGF-β signaling.

A variety of TGF-β antagonists and methods for their production are well known in the art and many more are currently under development (see for example, Dennis & Demetriou, 1998). The specific TGF-β antagonist employed is not a limiting feature; any effective TGF-β antagonist as defined herein may be useful in the methods and compositions of this disclosure. Preferably, the TGF-β antagonist is a TGF-β1, TGF-β2, or TGF-β3 antagonist. Most preferably the antagonist is a TGF-β1 antagonist.

Examples of TGF-β antagonists include, but are not limited to: monoclonal and polyclonal antibodies directed against one or more isoforms of TGF-β (Dasch, et al., 1996; Thompson et al., 1997 & 2000); TGF-β receptors or antibodies directed against TGF-β receptors (Segarini et al., 1997; Lin et al., 1999a; Lin et al., 1999b; Lin et al., 2000; Iwata et al., 1992; Lin et al., 1993; Ruoslahti et al., 1995; and Gotwals et al., 1998); latency associated peptide (Levinson et al., 1991); large latent TGF-β (Heldin et al., 1994); fetuin (Dennis and Demetriou, 1998); decorin and other proteoglycans such as biglycan, fibromodulin, lumican and endoglin (Ruoslahti and Yamaguchi., 1996; Ruoslahti et al., 1997; Ruoslahti et al., 1998; Ruoslahti et al., 1998; Border, 1998; Letarte et al., 1998; Letarte et al., 2000; Border and Ruoslahti, 1991; Ruoslahti and Yamaguchi, 1991; Ruoslahti et al., 1993; and Letarte et al., 1994); somatostatin (Culler and Kasprzyk, 1998); mannose-6-phosphate or mannose-1-phosphate (Ferguson, 1996); prolactin (Mcpherson and Richards, 1997); insulin-like growth factor II (Jeffrey and Gosiewska, 1998); IP-10 (Luster and Leder, 1997); arg-gly-asp containing peptides (Pfeffer, 1999; Ruoslahti and Border, 1993); extracts of plants, fungi and bacteria (Aoki et al., 1993; Mayumi et al., 1996; and Matsunaga et al., 1997); antisense oligonucleotides (Chung, 1997; Fakhrai et al., 1998; Dzau, 1998; Dzau, 1999; and Schlingensiepen et al., 1994); proteins involved in TGF-β signaling, including SMADs and MADs (Okazaki and Kitamura, 1998; Donahoe and Wang, 1997; Goldstein, 1997; Matsumoto and Irie, 1998; Ni et al., 1998; Wrana et al., 1998; Gimeno and Falb, 1998; Nakao et al., 1998; Verschueren et al., 1998; Miyazono and Kawabata, 1998; Whitman and Chen, 1998; Grinnell et al., 1999; Falb, 1998; Falb and Gimeno, 1998; and Gimeno and Falb, 1999), Ski and Sno (Vogel, 1999; and Stroschein et al., 1999); and any mutants, fragments or derivatives of the above-identified molecules that retain the ability to inhibit the activity of TGF-β.

In a preferred instance of this disclosure, e.g. in a preferred embodiment, the TGF-β antagonist is a human or humanized monoclonal antibody that blocks TGF-β binding to its receptor, or is selected from fragments thereof such as F(ab)₂ fragments, Fv fragments, single chain antibodies and other forms of "antibodies" that retain the ability to bind to TGF-β. In one instance, e.g. in one embodiment, the TGF-β antagonist is a human antibody produced by phage display (Thompson et al., 2000). In a more preferred instance, e.g. a more preferred embodiment, the monoclonal antibody is a humanized form of the murine monoclonal antibody obtained from hybridoma 1D11.16 (ATCC Accession No. HB 9849, as described in Dasch, et al., 1996, 1998a, and 1998b).

Mutants, variants, derivatives and analogues of the aforementioned TGF-β antagonist may also be useful in the methods of this disclosure. As used herein, "mutants, variants, derivatives and analogues" refer to molecules with similar shape or structure to the parent compound and that retain the ability to act as TGF-β antagonists. For example, any of the TGF-β antagonists disclosed herein may be crystallized, and useful analogues may be rationally designed based on the coordinates responsible for the shape of the active site(s). Alternatively, the ordinarily skilled artisan may, without undue experimentation, modify the functional groups of a known antagonist and screen such modified molecules for increased activity, half-life, bioavailability or other desirable characteristics. Where the TGF-β antagonist is a polypeptide, fragments and modifications of the polypeptide may be produced to increase the ease of delivery, activity, half life, etc (for example, humanized antibodies or functional antibody fragments, as discussed above). Given the level of skill in the art of synthetic and recombinant polypeptide production, such modifications may be achieved without undue experimentation.

Having now described the present invention in detail, the same will be more clearly understood by reference to the following examples, which are included for purposes of illustration.

### EXAMPLE 1: A High Salt Diet Dahl S Rat Animal Model Treated with a TGF-β Antagonist

Studies indicate that the renal production of TGF-β may be stimulated by elevations in dietary salt intake (Ying and Sanders, 1998b; Yu et al, 1998; Tamaki et al., 1996). To study the prophylactic effects of a TGF-β antagonist on the development of hypertension and renal dysfunction, Dahl S rats were fed a high salt (8.0% NaCl) diet as an animal model for hypertension and renal injury. As discussed earlier, the Dahl salt-sensitive (Dahl S) rat, which develops hypertension and renal injuries when challenged with a high salt diet, is well known and used in the art as a model for chronic renal disorders (see for example Karlsen et al., 1997).

Male Dahl SS/Jr rats (obtained from a colony maintained at the Medical College of Wisconsin) were fed a low salt diet (0.1% NaCl) until nine weeks of age to maintain a normal blood pressure and minimize renal injury.

At nine weeks of age the animals (∼250 to 300 g) were switched to a high salt (8% NaCl) diet for three weeks (water provided *ad libitum*). At ten weeks old the animals were divided into three treatment groups: group I animals received i.p. injections of anti-TGF-β antibody 1D11.16 (Genzyme Corporation, Framingham, MA), at a dose of 5.0 mg/Kg every other day for two weeks; group II animals received a 0.5 mg/Kg dose of anti-TGF-β antibody 1D11 every other day for two weeks; and a control group of animals received a 5.0 mg/Kg dose of a control antibody every other day for two weeks. The control antibody was an anti-verotoxin murine monoclonal IgG1 antibody (MAb 13C4, Genzyme Corporation, Framingham, MA). For the purposes of statistical analysis, data from the two test groups were pooled and compared to the control group.

### EXAMPLE 2: Effects of a TGF-β Antagonist on Mean Arterial Pressure (MAP)

To demonstrate the effectiveness of a TGF-β antagonist in maintaining appropriate systemic blood pressure in a hypertensive-induced animal model, arterial pressure of the Dahl S rats subjected to the treatment regime described in Example 1 was monitored.

During week two of the high salt diet, subject animals were anesthetized with an i.m. injection of ketamine (40 mg/kg), xylazine (2.5 mg/kg), and acepromazine (0.6 mg/Kg). An indwelling catheter was inserted into the femoral artery for continuous measurement of mean arterial pressure (MAP). The catheter was tunneled subcutaneously to the back of the neck, fed through a Dacron-mesh button sutured beneath the skin, advanced through a stainless steel spring that was connected to a swivel (Instech Laboratories, Plymouth Meeting, PA) and mounted above the animal's cage to permit free movement of the animals within their cages.

Animals recovered from surgery for one week. During week three, MAP and heart rate (HR) of the conscious and active animals were recorded (at a sample rate of 100 HZ) daily for four consecutive days between 1:00 and 5:00 PM. Heart rate, systolic, diastolic, and mean arterial pressures were averaged over 1 min intervals. Individual mean daily values were calculated. Daily averages were used to calculate an overall average value for the 4-day recording period for each animal.

Mean values (± 1 SEM) were calculated. The significance of differences in mean values between control and anti-TGF-β antibody treated groups was analyzed using an analysis of variance followed by a Duncan's multiple-range test. A P value< 0.05 was considered statistically significant.

Control group MAP averaged 190 ± 4 mm Hg (n=12). There was no significant difference in MAP of animals treated with low-dose (0.5 mg/Kg) versus high-dose (5.0 mg/Kg) anti-TGFβ antibody. Overall MAP averaged 177 ± 3 mm Hg (n=17) in the in the test group. MAP of test animals was significantly lower than the MAP of control animals.

These results demonstrate that treatment with a TGF-β antagonist significantly reduces hypertension in a hypertensive animal model.

### EXAMPLE 3: Measurement of Proteinuria and Plasma and Urinary Creatinine Clearance

To demonstrate the effects of anti-TGF-β treatment on urinary protein clearance in an animal model subjected to renal insult, overnight urine samples were collected from Dahl S rats subjected to the treatment regime described in Example 1 at the end of week three of the 3-week high salt diet, and the samples assayed for proteinuria and urinary creatinine clearance.

Mean values (± 1 SEM) were calculated. The significance of differences in mean values between control and anti-TGF-β antibody treated groups was analyzed using an analysis of variance followed by a Duncan's multiple-range test. A P value< 0.05 was considered statistically significant.

Proteinuria in control animals averaged 226 ± 20 mg/day (n=12). Proteinuria in animals treated with anti-TGF-β antibody, however, averaged 154 ± 16 mg/day (n=20); significantly lower than that of the control group.

In contrast, no significant difference was observed in plasma creatinine concentration and urinary creatinine clearance between experimental and control groups. Plasma creatinine concentration averaged 0.9 ± 0.15 mg/dl (n=11) for the control group, and averaged 1.28 ± 0.23 (n=16) for anti-TGF-β antibody treated animals. Both values are elevated compared to normal values of 0.52± 0.06 mg/dl (n=16) measured in a control group of salt resistant Brown Norway rats fed a high salt diet for three weeks. Creatinine clearance averaged 0.40 ± 0.08 ml/min/Kg-wt (n=7) for control animals, and 0.35 ± 0.07 ml/min/Kg-wt (n=8) for animals treated with the anti-TGF-β antibody.

These results demonstrate the effectiveness of a TGF-β antagonist to significantly reduce urinary protein excretion associated with kidney damage *in vivo.*

Despite the significant reduction in urinary protein excretion, indices of glomerular function (plasma creatinine concentration and creatinine clearance) were not significantly different between the control group and the TGFβ antagonist treated group.

These results are highly unexpected in view of current scientific evidence correlating enhanced TGF-β expression and glomerular extracellular matrix in diabetic rats (Sharma & Ziyadeh, 1994), normotensive rats (Yamamoto et al., 1994), transgenic mice that overexpress TGF-β (Kopp et al., 1991), and Dahl S rats (Tamaki et al., 1996). The present invention demonstrates for the first time that TGFβ antagonists are useful to maintain renal (medullary) function, separate and distinct from any potential therapeutic effect of TGFβ inhibitors on glomeruloscierosis (as suggested in the art).

### EXAMPLE 4: Effects of a TGF-β Antagonist on Renal Vascular and Tubular Flow

To demonstrate the effectiveness of a TGF-β antagonist in maintaining appropriate kidney function in a renally stressed animal model, renal blood flow (RBF), cortical blood flow (CBF), medullary blood flow (MBF), and glomerular flow rate (GFR) was monitored in Dahl S rats subjected to the treatment regime described in Example 1.

At the end of week three of the 3-week high salt diet, study animals were anesthetized with an i.m. injection of ketamine (30 mg/kg) and an i.p. injection of thiobarbituric acid (Inactin, 50 mg/kg)., Individuals were placed on a thermostatically controlled warming table (to maintain 37° C body temperature throughout the experimental procedure) and received an i.v. infusion of a 0.9 % NaCl solution containing 1% bovine serum albumin at a rate of 6 ml/h. [³H]-inulin (2 µCi/ml) was included in the infusion solution to permit quantification of inulin clearance as one measurement of GFR (in addition to creatinine clearance).

After tracheostomy, a cannula was inserted into the external jugular vein for i.v. infusions and into the femoral artery for arterial pressure measurement. The left ureter was cannulated for collection of urine. A 2 mm flow probe was positioned around the left renal artery to measure RBF using an electromagnetic flowmeter (Carolina Instruments, King, NC). Following the surgical procedure, subjects were stabilized for one hour prior to urine flow and plasma [³H]-inulin concentration sampling.

After the equilibration period, urine and plasma samples were collected during two separate consecutive 20-minute clearance periods to measure GFR and RBF. Blood pressure was also recorded.

After the second clearance period, CBF was measured from 5 sites on the renal cortex using an external probe (PF 316) and a laser Doppler flowmeter (Pf3, Perimed Corp., Stockholm, Sweden). MBF was measured using an acutely implanted fiber optic probe (as described in Mattson, 1993; and Kelly et al. 1999).

Mean values (± 1 SEM) were calculated. The significance of differences in mean values between control and anti-TGF-β antibody treated groups was analyzed using an analysis of variance followed by a Duncan's multiple-range test. A P value< 0.05 was considered statistically significant.

MAP averaged 160 ± 14 mm Hg after anesthesia for control animals. MAP was significantly lower in anti-TGFβ-antibody treated animals, however, averaging 145 ± 12 mm Hg after anesthesia.

RBF averaged 3.13 ± 0.67 ml/min/Kg-wt (n=7) for control animals, and 3.22 ± 0.41 ml/min/Kg-wt (n=14) for anti-TGFβ-antibody treated animals. The data demonstrate an 3% increase in renal blood flow in animals treated with a TGF-β antagonist compared to the control group; the difference between control and test group averages were not statistically significant.

Although the CBF signal was 22% higher in treated animals compared to the control group, the difference between anti-TGF-β antibody treated animals (1.85 ± 0.23 volts; n=13) and control animals (2.26 ± 0.19 volts; n=10) was not statistically significant.

More specifically, inulin clearance (as a separate measure of GFR in addition to creatinine clearance reported in Example 3), although 17% higher in test versus control groups, were not statistically significant. Inulin clearance in animals receiving TGFβ-antagonist treatment averaged 0.47 ± 0.03 ml/min/Kg-wt. inulin clearance in control animals averaged 0.52 ± 0.10 ml/min/Kg-wt.

In notable contrast, however, MBF was significantly (154%) higher in anti-TGF-β antibody treated animals (0.99 ± 0.12; n=13) compared to MBF in control animals (0.39 ± 0.09; n=10).

These results demonstrate for the first time the effectiveness of TGF-β therapy in maintaining renal medullary function. These findings further support the present discovery that therapeutic treatment with a TGF-β antagonist is useful to reduce hypoxic tubular injury, thus reducing proteinuria independent of any effect on glomerular function or structure, and further suggest that preservation of medullary blood flow contributes to the antihypertensive effect of TGF-β therapy (demonstrated in Example 2).

### EXAMPLE 5: Effects of a TGF-β Antagonist on Renal Structure

To examine the effects of a TGF-β antagonist in maintaining renal structural integrity in a renally-stressed animal model, gross and histological kidney analyses, as well as molecular assays, were performed on the kidneys of the Dahl S rats subjected to the treatment regime described in Example 1.

At the end of the 12-week experimental protocol outlined in Example 1, subject kidneys were harvested and weighed. The right kidney of each animal was frozen in liquid nitrogen and stored at - 80° C for measurement of collagen type III, fibronectin, TGF-β1 and TGF-β2 mRNA levels. The left kidney was fixed with 5% buffered formalin solution, embedded in paraffin, sectioned and stained with PAS for light microscopy analyses.

Glomerular diameters were measured and the degree of matrix expansion and glomerular injury was assessed on a minimum of 20 glomeruli/section. The degree of glomerulosclerosis was scored as previously described by Raij et al. (1984). The percentage of glomerular capillary area filled in with matrix was recorded on a scale from 0-4. A score of 0 indicates no damage; a score of 2 indicates 50% of the glomerular capillary area is obliterated; and a score of 4 indicates complete (100%) closure of all the capillaries within a given glomerulus.

Histological sections were also examined (light microscopic sections stained with PAS) for fibrosis of vasa recta capillaries and the degree of tubulointerstitial damage.

Average kidney weights between control (1.87 ± 0.06 g; n=12) and anti-TGF-β antibody treated (1.76 ± 0.06 g; n=20) animals were near identical, indicating that the degree of renal hypertrophy in the two groups was similar.

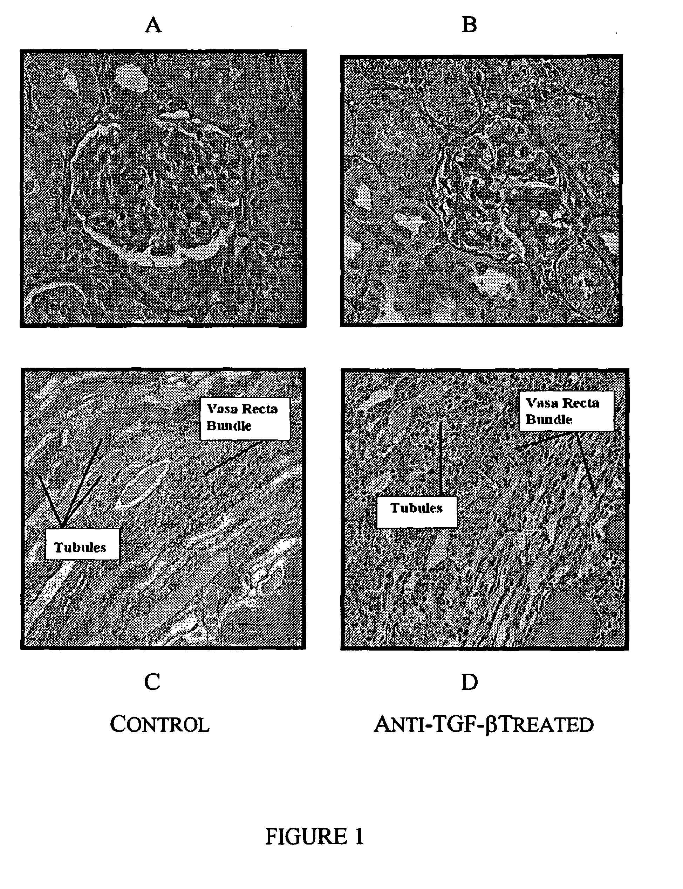

The effect of blocking TGF-β function on glomerular morphology is illustrated by the representative PAS-stained kidney sections presented in Figure 1 (panels A & B). Histological examination of kidneys from treated and untreated groups revealed that there was marked expansion of mesangial matrix in nearly every glomeruli. A large percentage (50-75%) of glomerular capillaries were filled in with matrix material with PAS-positive staining in most of the more severely injured glomeruli.

Anti-TGF-β antibody treatment had no effect on mean glomerular diameter (123.3 ± 1.4 µm) compared to control treatment (121.2 ± 2.3 µm). In addition, anti-TGF-β antibody treatment had no significant effect on the degree of glomerular injury. FGS score averaged 2.8 ± 0.18 (70% damage; n=176 glomeruli from 8 animals) in treated animals compared to 2.5 ± 0.11 (63% damage; n=140 glomeruli from 7 animals) in control animals.

These results prompted an examination of the degree of glomerular injury in a control group of normotensive Dahl S fed a low salt (0.1% NaCl) diet. There was no significant difference in FGS scores seen in control group; the treated group, and the group maintained on a low salt diet (however, mean glomerular diameter and kidney weight was lower, and the degree of proteinuria was also markedly reduced, in the low salt diet group).

These findings indicate that Dahl S rats spontaneously develop renal disease and that hypertension accelerates proteinuria and renal hypertrophy, but it does not increase the severity of glomerular injury. Comparative histological examination suggests that glomerular lesions may exist prior to dietary salt challenge in Dahl S rats, and calls into question the association between glomerular injury and hypertension.

In stark contrast to the above observations of cortical injury, histological examination of the renal medulla revealed reduced injury and damage in animals treated with the TGF-β antagonist compared to control animals. Panels C and D of Figure 1 are representative of renal medulla histological sections prepared from untreated and anti-TGF-β treated animals. Renal sections taken from anti-TGF-β treated animals revealed minimal affect to the medulla, with no evidence of capillary damage or tubular necrosis of the thick ascending limbs. In renal sections taken from control animals, however, vasa recta bundles were fibrotic and the capillaries completely occluded. In addition, renal sections from control animals revealed that the thick ascending limbs surrounding the capillaries were necrotic and tubule lumens were filled with protein casts.

Although anti-TGF-β antibody effectively reduced urinary protein excretion by 50% (Example 3, above), histological examination failed to reveal any improvement in the degree of glomerular injury or hypertrophy of the glomerulus or cortex. These results demonstrate the renoprotective effects of TGF-β antagonist therapy by preventing medullary hypoxic injury associated with reduced blood flow to the renal medulla.

### EXAMPLE 6: Effects of a TGF-β Antagonist on mRNA Expression Levels

To examine the effects of a TGF-β antagonist on the cellular expression of key proteins in a renally-stressed animal model, mRNA expression levels of collagen type III, fibronectin, TGF-β1 and TGF-β2 in the kidneys of the Dahl S rats subjected to the treatment regime described in Example 1 was quantified following ribonuclease protection assay (RPA) protocols known in the art.

To generate RNA probes useful for RPA (i.e., riboprobes) for each assay target (i.e., collagen type III, fibronectin, TGF-β1 and TGF-β2 mRNA's), reverse transcription polymerase chain reaction (RT-PCR) protocols well known in the art were employed to generate cDNA's (from total cellular RNA) using synthesized oligonucleotide primers complementary to each of the target sequences to be assayed (See Odermatt et al., 1985, for fibronectin cDNA sequence; Glumoff et al., 1994, for EDA domain of Fibronectin, and Collagen Type III cDNA sequence; Derynck et al., 1985, for TGF-β 1 cDNA sequence; and Madisen et al., 1988, for TGF-β2 cDNA sequence).

Linearized cDNA was transcribed *in vitro* using the MAXIscript™ *in vitro* transcription kit (Ambion, Inc., Austin, TX) according to the manufacturer's instructions. T7 polymerase and P³²CTP (3,000Ci/mmol; Dupont-NEN, Boston, MA) were included in the reaction mixture to generate P³² labeled riboprobe. The reaction mixture was incubated at 37° C for 60 min., then the DNA template was removed by digestion with 0.5U RNase-free DNase. Full length probes were purified from the transcription reaction by electrophoresis on 6% polyacrylamide /TBE gel. Bands were identified by autoradiography, each (antisense) RNA probe was excised from the gel band, and passively diffused into probe elution buffer MAXIscript™ kit; Ambion, Inc., Austin, TX) at 37° C overnight. The activity of each probe was quantified by scintillation counting.

At the end of the 12-week experimental protocol outlined in Example 1, subject kidneys were harvested and weighed. The right kidney of each animal was frozen in liquid nitrogen and stored at - 80° C for assay of collagen type III, fibronectin, TGF-β1 and TGF-β2 mRNA levels.

Total cellular RNA from whole kidney tissue was obtained using the RNAqueous™ kit for purification of total RNA (Ambion, Inc., Austin, TX) according to the manufacturer's instructions. RPA was performed using the HybSpeed™ RPA kit (Ambion, Inc., Austin, TX) according to the manufacturer's instructions. Briefly, radiolabeled antisense RNA probe for fibronectin, collagen type III, TGF-β1 and TGF-β2 were combined and hybridized with 10µg of total cellular RNA from different kidney samples. An 18S RNA probe (Ambion, Inc., Austin, TX) was also included in each hybridization mixture to normalize total RNA in individual samples. RPA was also performed on yeast RNA as a negative control. Digestion with RNaseA/RNaseT 1 mix was performed to degrade unhybridized RNA's. Hybridized RNA, protected from digestion, were resolved by electrophoresis and visualized by using a Fujifilm BAS-1500 phosphoimager (Fujifilm, Tokyo, JP). Phosphoimager bands representing collagen type III, fibronectin, TGF-β1 and TGF-β2 cellular mRNA were quantified using MacBAS Version 2.4 software. Data obtained from each sample were standardized.

Collagen type III and fibronectin mRNA expression levels were markedly reduced in the Dahl S rats treated with the anti-TGF-β Ab (relative intensity 38.5 ± 7.5 and 38.9 ± 4.5 respectively; n=13) compared to the control group (relative intensity 100 ± 7.5 and 100 ± 4.5 respectively; n=5).

TGF-β 1 and TGF-β2 mRNA expression levels were also markedly reduced in the Dahl S rats treated with the anti-TGF-β Ab (relative intensity 0.08 ± 0.01 and 0.07 ± 0.01 respectively; n=13) compared to the control group (relative intensity 0.26 ± 0.04 and 0.30 ± 0.02 respectively; n=5). These results likely reflect less renal fibrosis and are consistent with the collagen type III and fibronectin mRNA expression profiles.

### EXAMPLE 7: Early Intervention Effects of TGF-β Antagonist Therapy

To demonstrate the renoprotective effectiveness of early intervention TGF-β antagonist therapy, 6 week old male Dahl S rats fed a high salt diet were subjected to an anti-TGF-β-Ab (0.5 mg/day) treatment regime similar to that described in Example 1 for three weeks.

At six weeks of age (as opposed to nine weeks as described in Example 1), Dahl S rats (n= 22, 175 to 200 g) were switched to a high salt (8% NaCl) diet for three weeks (water provided *ad libitum*). Test treatment also started at six weeks of age (as opposed to ten weeks as described in Example 1). The animals were randomly divided into two experimental groups: the treatment group received i.p. injections of anti-TGF-β Ab at a dose of 0.50 mg/kg every other day for three weeks; the control group received i.p. injections of the control MAb (MAb 13C4, Genzyme Corporation, Framingham, MA) every other day for three weeks.

### EXAMPLE 7.1: Early Intervention Measurement of Proteinuria and Microalbuminuria

To demonstrate early intervention effectiveness of TGF-β antagonist therapy on urinary protein clearance in an animal model subjected to renal insult, urine samples were collected from Dahl S rats subjected to the treatment regime described in Example 7 on days 4, 11, 18, and 21 of the high salt diet, and the samples assayed for proteinuria and urinary albumin clearance. An overnight urine sample was also collected before the animals were switched to the high salt diet as a baseline data point.

Proteinuria was determined using the Bradford method (Bio-Rad Laboratories Hercules, CA) with bovine serum albumin as the standard. Urine albumin concentration was determined by the Albumin Blue 580 method (Molecular Probes, Eugene, OR).

Mean values (± 1 SEM) were calculated. The significance of differences in mean values between control and anti-TGF-β antibody treated groups was analyzed using an analysis of variance followed by a Duncan's multiple-range test. A P value< 0.05 was considered statistically significant.

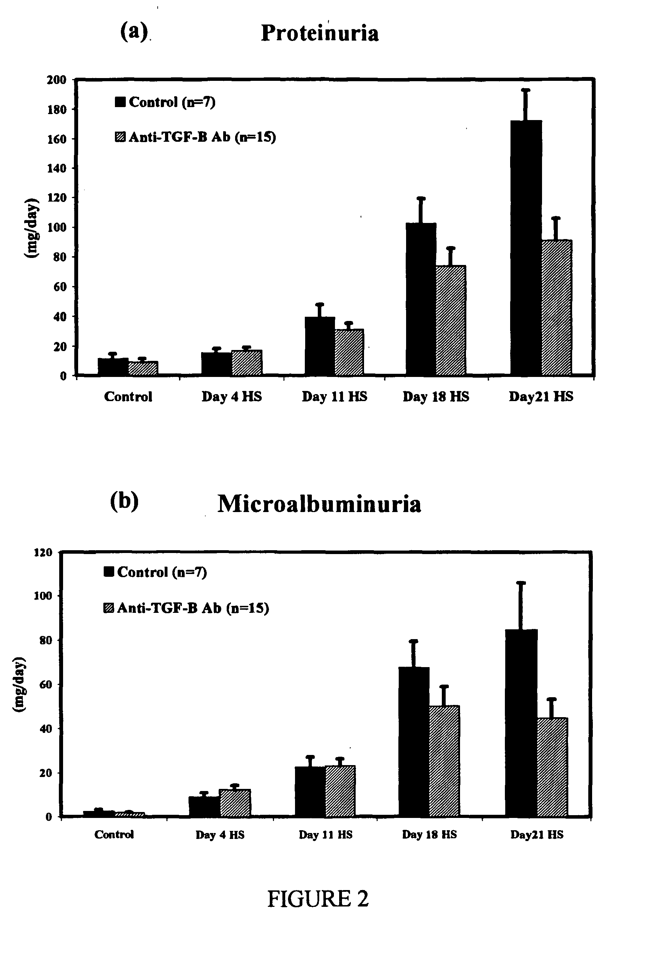

The results are presented in Figure 2.

Proteinuria was under 20 mg/day in both the anti-TGF-β Ab treated and control Dahl S rats fed a low salt (0.1%) diet. Protein excretion gradually increased in both the control and anti-TGF-β Ab treated rats during the first two weeks of a high salt diet. By day 18 of the high salt diet, severe proteinuria was observed in both experimental groups. The degree of proteinuria tended to be lower in the anti-TGF-β Ab treated group (74.2 ± 11.6 mg/day; n=15) than in the control group (102.7 ± 16.8 mg/day; n=7), however. After three weeks on a high salt diet, the severity of the proteinuria in Dahl S rats was significantly less in the anti-TGF-β Ab treated animals (91 ± 20 mg/day) compared to the control group (172 ± 20 mg/day) (Fig. 2a).

The effect of anti-TGF-β Ab treatment on albumin excretion in test animals was similar (Fig. 2b). In both control and anti-TGF-β Ab treated animals fed a low salt diet (0.1% NaCl), albumin excretion was under 10 mg/day. After three weeks on a high salt diet, however, albumin excretion was significantly lower in the urine of Dahl S rats treated with anti-TGF-β Ab (44.8 ± 8.4 mg/day; n=15) than in the control rats (84.6 ± 21.4 mg/day; n=7).

These results demonstrate the effectiveness of early intervention TGF-β antagonist therapy to significantly reduce urinary protein and albumin excretion associated with kidney damage *in vivo.*

### EXAMPLE 7.2: Early Intervention Effects of a TGF-β Antagonist on Renal Structure

To examine the early intervention effects of a TGF-β antagonist in maintaining renal structural integrity in a renally-stressed animal model, gross and histological kidney analyses, as well as molecular assays, were performed on the kidneys of the Dahl S rats subjected to the treatment regime described in Example 7 following protocols described in Example 5.

Histological examination of harvested kidneys revealed that a large percentage of glomerular capillaries were filled in with matrix material, with PAS-positive material in most of the severely injured glomeruli. Anti-TGF-β Ab treatment prior to initiating the high salt diet in younger Dahl S rats reduced the degree of glomerular injury, however. Focal glomerulosclerosis injury score averaged 3.25 ± 0.06 (n=144 glomeruli from 7 animals) in Dahl S rats treated with the control Ab. In contrast, glomerular injury in anti-TGF-β Ab treated was a significantly lower (2.73 ± 0.04; n=382 glomeruli from 15 animals).

Anti-TGF-β Ab therapy also reduced the degree of fibrosis of vasa recta capillaries, necrosis of thick ascending loop of Henle and formation of protein casts. In the control group, 22.2 ± 1.3% (n=7) of the area in the outer medulla was filled with protein casts compared to 5.57 ± 0.32 % (n=15) in anti-TGF-β Ab treated animals; representing a four-fold decrease in the degree of protein cast formation.

The pratice of the present invention may involve techniques well known in the field of molecular biology. These techniques include, but are not limited to, techniques described in the following publications:
Ausubel, F.M. et al. eds., Short Protocols In Molecular Biology (4th. Ed. 1999) John Wiley & Sons, NY. (ISBN 0-471-32938-X).
Old, R.W. & S.B. Primrose, Principles of Gene Manipulation: An Introduction To Genetic Engineering (3d Ed. 1985) Blackwell Scientific Publications, Boston. Studies in Microbiology, V.2:409 pp. (ISBN 0-632-01318-4).
Sambrook, J. et al. eds., Molecular Cloning: A Laboratory Manual (2d Ed. 1989) Cold Spring Harbor Laboratory Press, NY. Vols. 1-3 (ISBN 0-87969-309-6).
Winnacker, E.L. From Genes To Clones; Introduction To Gene Technology (1987) VCH Publishers, NY (transtated by Horst Ibelgaufts). 634 pp. (ISBN 0-89573-614-4).

### References

Ando et al., 1998. Miner. Electrolyte and Metab. 24(2-3):149-153.
Aoki et al., 1993. EU 813875.
Basile & Hammerman, 1998. Miner. Electrolyte and Metab. 24(2-3):144-148.
Border et al., 1990. Nature. 346:371-374.
Border & Ruoslahti, 1991. WO 91/04748.
Border et al., 1992a, Nature. 360:361-364.
Border et al, 1992b. J. Clin. Invest. 90:1-7.
Border & Noble., 1994. NEJM, 331(19):1286-1292.
Border, 1996. WO 96/25178.
Border & Noble., 1998. Hypertension. 3:181-188.
Border, 1998. US PAT NO. 5,824,655.
Bottinger et al., 1997. Kidney Intern'l 51:1255-1360.
Bottinger & Kopp, 1998. Miner. Electrolyte and Metab. 24(2-3):154-160.
Brezis & Rosen, 1995. New Engl. J. Med. 332(10):647-655.
Brezis et al., 1991, J. Clin. Invest. 88:390-395.
Brezis et al., 1994a, Am. J. Physiol. 267:F1059-F1062.
Brezis et al., 1994b, Am. J. Physiol. 267:F1063-F1068.
Chung, 1997. US PAT NO. 5,683,988.
Culler and Kasprzyk, 1998. WO 98/08529.
Dasch, et al., 1996. US PAT NO. 5,571,714.
Dasch, et al., 1998a. US PAT NO. 5,772,998.
Dasch, et al., 1998b. US PAT NO. 5,783,185.
Dennis & Demetriou, 1998. US PAT NO. 5,821,227.
Derynck et al., 1985. Nature. 316(6030):701-705.
Donahoe and Wang, 1997. WO 97/31020.
Douthwaite et al., 1999. J. Am. Soc. Nephrol. 10:2109-2119.
Dzau, 1998. US PAT NO. 5,821,234.
Dzau, 1999. US PAT NO. 5,869,462.
Eddy, 1996. J. Am. Soc. Nephol. 7:2495-2508.
Eddy, 1994. J. Am. Soc. Nephol. 5:1273-1287.
English et al., 1987 Transplantation 44:135-141.
Fakhrai et al., 1998. US PAT NO. 5,772,995.
Falb, 1998. US PAT NO. 5,834,248.
Falb and Gimeno, 1998. US PAT NO. 5,807,708.
Gimeno and Falb, 1999. US PAT NO. 5,948,639.
Ferguson, 1996. US PAT NO. 5,520,926.
Ferguson et al., 1997. US PAT NO. 5,662,904.
Gimeno and Falb, 1998. WO 98/45467.
Glumoff et al., 1994. Biochim. Biophys. Acta. 1219(3):613-622.
Goldstein, 1997. WO 97/38729.
Gotwals et al., 1998. WO 98/48024.
Grande, 1997. Proc. Soc. Exp. Biol. Med. 214(1):27-40.
Grinnell et al., 1999. WO 99/50296.
Heldin et al., 1994. WO 94/09812.
Hocevar & Howe, 1998. Miner. Electrolyte and Metab. 24(2-3):131-135.
Isaka et al., 1993. J. Clin. Invest. 92:2597-2601.
Iwata et al., 1992. WO 92/00330.
Jeffrey and Gosiewska, 1998. WO 98/17304.
Johnson et al., 1997. Kidney Intern'l 52:1169-1179.
Johnson et al., 1999. Hypertension 34:151-159.
Karlsen et al., 1997 Hypertension 30:975-983.
Kelly et al., 1999. J. Am. Soc. Nephrol. 10:1264-1273, 1999.
Ketteler et al., 1994. Curr. Opin. Nephrol. and Hypertension 3:446-452.
Kopp et al., 1991. Lab. Invest. 74:991-1003.
Kuihara et al., 1989. Biochem. Biophys. Res. Comm. 159:1435-1440.
Letarte et al., 1994. WO 94/10187.
Letarte et al., 1998. US PAT NO. 5,830,847.
Letarte et al., 2000. US PAT NO. 6,015,693.
Levinson et al., 1991. WO 91/08291.
Li et al., 1999. Hypertension 33: 271-275.
Lin et al., 1993. WO 93/09228.
Lin et al., 1999a. US PAT NO. 6,001,969.
Lin et al., 1999b. US PAT NO. 6,008,011.
Lin et al., 2000. US PAT NO. 6,010,872.
Logan & Baird, 1999. US PAT NO. 5,958,411.
Luster and Leder, 1997. WO 97/00691.
Madisen et al., 1988. DNA. 7(1):1-8.
Mackay et al., 1992. J. Biol. Chem. 267:11449-11454.
Matsumoto and Irie, 1998. WO 98/03663.
Matsunaga et al., 1997. US PAT NO. 5,693,610.
Mattson, 1993. Am. J. Physiol. 264:H190-H195.
Mayumi et al., 1996. JP 8119984.
Mcpherson and Richards, 1997. WO 97/40848.
Miyazono and Kawabata, 1998. WO 98/56913.
Mozes et al., 1999. J. Am. Soc. Nephrol. 10:271-280.
Nabel et al. 1993. PNAS USA 90:10759-10763.
Nakao et al., 1998. WO 98/53068.
Ni et al., 1998. WO 98/07735.
Odermatt et al., 1985. PNAS USA 82:6571-6575.
Ohno et al., 1995. J. Clin. Invest. 95:1363-1369.
Okazaki and Kitamura, 1998. EP 874046.
Parrella et al., 1998. Miner. Electrolyte and Metab. 24(2-3):136-143.
Pfeffer, 1999. US PAT NO. 5,958,411.
Raij et al. 1984. Kidney Int. 26:137-143.
Roberts, 1998. Miner. Electrolyte and Metab. 24(2-3):111-119.
Ruiz-Torres, 1998. J. Am. Soc. Nephol. 9(5):782-791.
Ruoslahti and Yamaguchi., 1991. WO 91/10727.
Ruoslahti et al., 1993. WO 93/09800.
Ruoslahti & Border, 1993. WO 93/10808.
Ruoslahti et al., 1995. WO 95/10610.
Ruoslahti & Yamaguchi., 1996. US PAT NO. 5,583,103.
Ruoslahti et al., 1997. US PAT NO. 5,654,270.
Ruoslahti et al., 1998. US PAT NO. 5,705,609.
Ruoslahti et al., 1998. US PAT NO. 5,726,149.
Segarini et al., 1997. US PAT NO. 5,693,607.
Sharma & Ziyadeh, 1994. Am. J. Physiol. 267:F1094-F1101..
St. Lezin et al., 1994. Hypertension 23:786-790.
Sarzani et al., 1989. J. Clin. Invest. 83:1404-1408.
Schlingensiepen et al., 1994. WO 94/25588.
Sterzel et al., 1988. Kidney Intern'l. 33:1119-1129.
Stroschein et al., 1999. Science 286:771-774.
Tamaki et al., 1996. J. Am. Soc. Nephrol. 7:2578-2589.
Thompson et al., 1997. WO 97/13844.
Thompson et al., 2000. WO 00/66631.
Verschueren et al., 1998. WO 98/55512.
Vodovotz et al., 1996. J. Experimental Med. 183:2337-2342.
Vogel, 1999. Science. 286:665.
Whitman and Chen, 1998. WO 98/53830.
Wolf, 1998. Miner. Electrolyte and Metab. 24(2-3):174-180.
Wrana, 1998. Miner. Electrolyte and Metab. 24(2-3):120-130.
Wrana et al., 1998. WO 98/07849.
Yamamoto et al., 1994. Kidney Intern'l. 45:916-927.
Ying & Sanders, 1998a. Am. J. Physiol. 274:F635-F641.
Ymg & Sanders, 1998b. Am. J. Physiol. 275:F18-F24.
Yu et al., 1998. Hypertension. 98:2621-2628.

## Claims

1. Use of a TGF-β antagonist for the preparation of a pharmaceutical composition for reducing systemic hypertension, maintaining medullary blood flow, reducing the loss of renal medullary function, or reducing hypoxic tubular injury, in an individual suffering from or potentially susceptible to loss of kidney function, wherein said TGF-β antagonist is an antibody or antibody fragment that is directed against one or more isoforms of TGF-β.

2. The use of Claim 1, wherein said pharmaceutical composition is for reducing the loss of renal medullary function in an individual suffering from or potentially susceptible to loss of kidney function.

3. The use of Claim 1, wherein said pharmaceutical composition is for reducing hypoxic tubular injury in an individual suffering from or potentially susceptible to loss of kidney function.

4. The use of Claim 1, wherein said pharmaceutical composition is for reducing systemic hypertension in an individual suffering from or potentially susceptible to loss of kidney function.

5. The use of Claim 1, wherein said pharmaceutical composition is for maintaining medullary blood flow in an individual suffering from or potentially susceptible to loss of kidney function.

6. The use of Claim 1, wherein said individual is suffering from hypertension.

7. Use of a TGF-β antagonist for the preparation of a pharmaceutical composition for treating or slowing the progression of kidney damage associated with a disease or disorder of the kidney selected from the group consisting of obstructive nephropathy, polycystic kidney disease, nephrosclerosis, and nephrocalcinosis, wherein said TGF-β antagonist is an antibody or antibody fragment that is directed against one or more isoforms of TGF-β.

8. The use of any one of Claims 1-7, wherein said TGF-β antagonist is a monoclonal antibody that is directed against one or more isoforms of TGF-β.

9. The use of Claim 8, wherein said monoclonal antibody is a human or humanized monoclonal antibody.

10. The use of any one of Claims 1-7, wherein said TGF-β antagonist is an antibody fragment that is directed against one or more isoforms of TGF-β.

11. The use of Claim 10, wherein said antibody fragment is selected from the group consisting of an F(ab)₂ fragment, an Fv fragment, and a single chain antibody.

12. The use of any one of Claims 1-7, wherein said TGF-β antagonist is a humanized form of the monoclonal antibody 1D11.16 as deposited with the ATCC under accession number HB9849.

13. A TGF-β antagonist, for use in reducing systemic hypertension, maintaining medullary blood flow, reducing the loss of renal medullary function, or reducing hypoxic tubular injury, in an individual suffering from or potentially susceptible to loss of kidney function, wherein said TGF-β antagonist is an antibody or antibody fragment that is directed against one or more isoforms of TGF-β.

14. A TGF-β antagonist, for use in treating or slowing the progression of kidney damage associated with a disease or disorder of the kidney selected from the group consisting of obstructive nephropathy, polycystic kidney disease, nephrosclerosis, and nephrocalcinosis, wherein said TGF-β antagonist is an antibody or antibody fragment that is directed against one or more isoforms of TGF-β.

15. The TGF-β antagonist of Claim 13 or 14, wherein said TGF-β antagonist is a monoclonal antibody that is directed against one or more isoforms of TGF-β.

16. The TGF-β antagonist of Claim 15, wherein said monoclonal antibody is a human or humanized monoclonal antibody.

17. The TGF-β antagonist of Claim 13 or 14, wherein said TGF-β antagonist is an antibody fragment that is directed against one or more isoforms of TGF-β.

18. The TGF-β antagonist of Claim 17, wherein said antibody fragment is selected from the group consisting of an F(ab)₂ fragment, an Fv fragment, and a single chain antibody.

19. The TGF-β antagonist of Claim 13 or 14, wherein said TGF-β antagonist is a humanized form of the monoclonal antibody 1D11.16 as deposited with the ATCC under accession number HB9849.

## Patentansprüche

1. Verwendung eines TGF-β-Antagonisten zur Herstellung einer pharmazeutischen Zusammensetzung zur Reduktion von systemischer Hypertonie, Aufrechterhaltung des medullären Blutflusses, Reduktion des Verlustes der Nierenmarkfunktion oder Reduktion der hypoxischen tubulären Verletzung in einem Individuum, das an einem Verlust der Nierenfunktion leidet oder potentiell dafür anfällig ist, wobei der TGF-β-Antagonist ein Antikörper oder Antikörperfragment ist, der/das gegen eine oder mehrere Isoformen von TGF-β gerichtet ist.

2. Verwendung nach Anspruch 1, wobei die pharmazeutische Zusammensetzung der Reduktion des Verlusts der Nierenmarkfunktion in einem Individuum dient, das an einem Verlust der Nierenfunktion leidet oder potentiell dafür anfällig ist.

3. Verwendung nach Anspruch 1, wobei die pharmazeutische Zusammensetzung der Reduktion der hypoxischen tubulären Verletzung in einem Individuum dient, das an einem Verlust der Nierenfunktion leidet oder potentiell dafür anfällig ist.

4. Verwendung nach Anspruch 1, wobei die pharmazeutische Zusammensetzung der Reduktion der systemischen Hypertonie in einem Individuum dient, das an einem Verlust der Nierenfunktion leidet oder potentiell dafür anfällig ist.

5. Verwendung nach Anspruch 1, wobei die pharmazeutische Zusammensetzung der Aufrechterhaltung des medullären Blutflusses in einem Individuum dient, das an einem Verlust der Nierenfunktion leidet oder potentiell dafür anfällig ist.

6. Verwendung nach Anspruch 1, wobei das Individuum an Hypertonie leidet.

7. Verwendung eines TGF-β-Antagonisten zur Herstellung einer pharmazeutischen Zusammensetzung zur Behandlung oder Verlangsamung des Fortschritts eines Nierenschadens, der mit einer Krankheit oder Störung der Niere einhergeht, ausgewählt aus der Gruppe, bestehend aus obstruktiver Nephropathie, polycystischer Nierenkrankheit, Nephrosklerose, und Nephrocalcinose, wobei der TGF-β-Antagonist ein Antikörper oder Antikörperfragment ist, der/das gegen eine oder mehrere Isoformen von TGF-β gerichtet ist.

8. Verwendung nach einem der Ansprüche 1 bis 7, wobei der TGF-β-Antagonist ein monoklonaler Antikörper ist, der gegen eine oder mehrere Isoformen von TGF-β gerichtet ist.

9. Verwendung nach Anspruch 8, wobei der monoklonale Antikörper ein humaner oder humanisierter monoklonaler Antikörper ist.

10. Verwendung nach einem der Ansprüche 1 bis 7, wobei der TGF-β-Antagonist ein Antikörperfragment ist, das gegen eine oder mehrere Isoformen von TGF-β gerichtet ist.

11. Verwendung nach Anspruch 10, wobei das Antikörperfragment ausgewählt ist aus der Gruppe, bestehend aus einem F(ab)₂-Fragment, einem Fv-Fragment, und einem Einzelketten-Antikörper.

12. Verwendung nach einem der Ansprüche 1 bis 7, wobei der TGF-β-Antagonist eine humanisierte Form des monoklonalen Antikörper 1D11.16 ist, wie er bei der ATCC unter der Zugangsnummer HB9849 hinterlegt ist.

13. TGF-β-Antagonist zur Verwendung bei der Reduktion der systemischen Hypertonie, Aufrechterhaltung des medullären Blutflusses, Reduktion des Verlustes der Nierenmarkfunktion oder Reduktion der hypoxischen tubulären Verletzung, in einem Individuum, das an einem Verlust der Nierenfunktion leidet oder potentiell dafür anfällig ist, wobei der TGF-β-Antagonist ein Antikörper oder Antikörperfragment ist, der/das gegen eine oder mehrere Isoformen von TGF-β gerichtet ist.

14. TGF-β-Antagonist zur Verwendung bei der Behandlung oder Verlangsamung des Fortschritts eines Nierenschadens, der mit einer Krankheit oder Störung der Niere einhergeht, ausgewählt aus der Gruppe, bestehend aus obstruktiver Nephropathie, polycystischer Nierenkrankheit, Nephrosklerose, und Nephrocalcinose, wobei der TGF-β-Antagonist ein Antikörper oder Antikörperfragment ist, der/das gegen eine oder mehrere Isoformen von TGF-β gerichtet ist.

15. TGF-β-Antagonist nach Anspruch 13 oder 14, wobei der TGF-β-Antagonist ein monoklonaler Antikörper ist, der gegen eine oder mehrere Isoformen von TGF-β gerichtet ist.

16. TGF-β-Antagonist nach Anspruch 15, wobei der monoklonale Antikörper ein humaner oder humanisierter monoklonaler Antikörper ist.

17. TGF-β-Antagonist nach Anspruch 13 oder 14, wobei der TGF-β-Antagonist ein Antikörperfragment ist, das gegen eine oder mehrere Isoformen von TGF-β gerichtet ist.

18. TGF-β-Antagonist nach Anspruch 17, wobei das Antikörperfragment ausgewählt ist aus der Gruppe, bestehend aus einem F(ab)₂-Fragment, einem Fv-Fragment, und einem Einzelketten-Antikörper.

19. TGF-β-Antagonist nach Anspruch 13 oder 14, wobei der TGF-β-Antagonist eine humanisierte Form des monoklonalen Antikörper 1D11.16 ist, wie er bei der ATCC unter der Zugangsnummer HB9849 hinterlegt ist.

## Revendications

1. Utilisation d'un antagoniste de TGF-β pour la préparation d'une composition pharmaceutique pour réduire l'hypertension systémique, maintenir la circulation sanguine médullaire, réduire la perte de fonction médullaire rénale, ou réduire une lésion tubulaire hypoxique, chez un individu souffrant de ou potentiellement susceptible de perte de fonction rénale, ledit antagoniste de TGF-β étant un anticorps ou fragment d'anticorps qui est dirigé contre une ou plusieurs isoformes de TGF-β.

2. Utilisation de la revendication 1, ladite composition pharmaceutique étant pour réduire la perte de fonction médullaire rénale chez un individu souffrant de ou potentiellement susceptible de perte de fonction rénale.

3. Utilisation de la revendication 1, ladite composition pharmaceutique étant pour réduire une lésion tubulaire hypoxique chez un individu souffrant de ou potentiellement susceptible de perte de fonction rénale.

4. Utilisation de la revendication 1, ladite composition pharmaceutique étant pour réduire l'hypertension systémique chez un individu souffrant de ou potentiellement susceptible de perte de fonction rénale.

5. Utilisation de la revendication 1, ladite composition pharmaceutique étant pour maintenir la circulation sanguine médullaire chez un individu souffrant de ou potentiellement susceptible de perte de fonction rénale.

6. Utilisation de la revendication 1, ledit individu souffrant d'hypertension.

7. Utilisation d'un antagoniste de TGF-β pour la préparation d'une composition pharmaceutique pour traiter ou ralentir la progression de dommages rénaux associés à une maladie ou un trouble des reins choisi dans le groupe constitué de la néphropathie obstructive, la maladie polykystique des reins, la néphrosclérose, et la néphrocalcinose, ledit antagoniste de TGF-β étant un anticorps ou fragment d'anticorps qui est dirigé contre une ou plusieurs isoformes de TGF-β.

8. Utilisation de l'une quelconque des revendications 1 à 7, ledit antagoniste de TGF-β étant un anticorps monoclonal qui est dirigé contre une ou plusieurs isoformes de TGF-β.

9. Utilisation de la revendication 8, ledit anticorps monoclonal étant un anticorps monoclonal humain ou humanisé.

10. Utilisation de l'une quelconque des revendications 1 à 7, ledit antagoniste de TGF-β étant un fragment d'anticorps qui est dirigé contre une ou plusieurs isoformes de TGF-β.

11. Utilisation de la revendication 10, ledit fragment d'anticorps étant choisi dans le groupe constitué d'un fragment F(ab)₂, un fragment Fv, et un anticorps monocaténaire.

12. Utilisation de l'une quelconque des revendications 1 à 7, ledit antagoniste de TGF-β étant une forme humanisée de l'anticorps monoclonal 1D11.16 tel que déposé à l'ATCC sous le numéro d'accession HB9849.

13. Antagoniste de TGF-β, pour utilisation dans la réduction de l'hypertension systémique, le maintien de la circulation sanguine médullaire, la réduction de la perte de fonction médullaire rénale, ou la réduction d'une lésion tubulaire hypoxique, chez un individu souffrant de ou potentiellement susceptible de perte de fonction rénale, ledit antagoniste de TGF-β étant un anticorps ou fragment d'anticorps qui est dirigé contre une ou plusieurs isoformes de TGF-β.

14. Antagoniste de TGF-β, pour utilisation dans le traitement ou le ralentissement de la progression de dommages rénaux associés à une maladie ou un trouble des reins choisi dans le groupe constitué de la néphropathie obstructive, la maladie polykystique des reins, la néphrosclérose, et la néphrocalcinose, ledit antagoniste de TGF-β étant un anticorps ou fragment d'anticorps qui est dirigé contre une ou plusieurs isoformes de TGF-β.

15. Antagoniste de TGF-β de la revendication 13 ou 14, ledit antagoniste de TGF-β étant un anticorps monoclonal qui est dirigé contre une ou plusieurs isoformes de TGF-β.

16. Antagoniste de TGF-β de la revendication 15, ledit anticorps monoclonal étant un anticorps monoclonal humain ou humanisé.

17. Antagoniste de TGF-β de la revendication 13 ou 14, ledit antagoniste de TGF-β étant un fragment d'anticorps qui est dirigé contre une ou plusieurs isoformes de TGF-β.

18. Antagoniste de TGF-β de la revendication 17, ledit fragment d'anticorps étant choisi dans le groupe constitué d'un fragment F(ab)₂, un fragment Fv, et un anticorps monocaténaire.

19. Antagoniste de TGF-β de la revendication 13 ou 14, ledit antagoniste de TGF-β étant une forme humanisée de l'anticorps monoclonal 1D11.16 tel que déposé à l'ATCC sous le numéro d'accession HB9849.
